# EUROPÄISCHE PATENTSCHRIFT

(11) **EP 2 445 867 B1**
(45) Veröffentlichungstag und Bekanntmachung des Hinweises auf die Patenterteilung: **12.09.2018**
(21) Anmeldenummer: 10727691.7
(22) Anmeldetag: 23.06.2010
(51) Int. Cl.: C07C 235/08, A61K 31/164

(54) **CERAMID-DIMERE UND IHRE VERWENDUNG ALS ARZNEIMITTEL ODER KOSMETISCHE ZUBEREITUNG**
CERAMIDE DIMERS AND THE USE THEREOF AS PHARMACEUTICAL PRODUCTS OR COSMETIC PREPARATION
DIMÈRES À BASE DE CÉRAMIDES ET LEUR UTILISATION EN TANT QUE MÉDICAMENT OU PRÉPARATION COSMÉTIQUE

(30) Priorität: 23.06.2009 EP 09008218
(43) Veröffentlichungstag der Anmeldung: 02.05.2012
(73) Patentinhaber: Wolf, Hans-Uwe, 89231 Neu-Ulm (DE)
(72) Erfinder: Wolf, Hans-Uwe, 89231 Neu-Ulm (DE)
(74) Vertreter: Pfenning, Meinig & Partner mbB
(86) Internationale Anmeldenummer: PCT/EP2010/003875
(87) Internationale Veröffentlichungsnummer: WO 2010/149383

(56) Entgegenhaltungen:
- WO-A1-2005/063688
- JP-A- H03 223 297
- JP-A- 2002 047 261
- YOSHIMI MUROZUKA ET AL: "Lyso-GM3, its dimer, and multimer: their synthesis, and their effect on epidermal growth factor-induced receptor tyrosine kinase", GLYCOCONJUGATE JOURNAL, KLUWER ACADEMIC PUBLISHERS, BO, vol. 24, no. 9, 19 July 2007 (2007-07-19), pages 551-563, XP019551594, ISSN: 1573-4986, DOI: 10.1007/S10719-007-9051-2

## Beschreibung

Die Erfindung betrifft Ceramid-Dimere, die aus zwei Ceramid-Molekülen aufgebaut sind, die über ihr lipophiles Ende miteinander verknüpft sind. Die Ceramid-Moleküle weisen dabei an ihrem hydrophilen Ende mindestens eine hydrophile Gruppe zur Vergrößerung der Hydrathülle des Dimers auf. Die erfindungsgemäßen Ceramid-Dimere können als Arzneimittel oder als kosmetische Zubereitung eingesetzt werden.

Grundsätzlich enthalten alle biologischen Membranen, insbesondere Zellmembranen, als essentielle Bestandteile sog. Lipide und Lipid-analoge Substanzen, die strukturell aus einem lipophilen (hydrophoben) und einem hydrophilen (lipophoben) Molekülteil aufgebaut sind, dass sie somit eine sog. amphiphile Struktur besitzen, die zum Teil sehr stark ausgeprägt ist.

Die amphiphile Struktur der Ceramide, d.h. die gleichzeitige Anwesenheit eines (stark) hydrophoben und eines hydrophilen, polaren Anteils der Molekülstruktur führt dazu, dass sich die Ceramide in einer wässrigen Phase (meist zusammen mit anderen Lipiden) spontan zu einer Lipid-Doppelschicht, einem so genannten "Lipid-Bilayer" anordnen, der u. a. die Grundlage der Struktur biologischer Membranen darstellt. Das Bauprinzip dieser Doppelschicht ist für Ceramide und alle anderen Lipide und Lipid-analogen Substanzen gleich: Sie ordnen sich in zwei parallelen, eng zusammenliegenden Schichten an, wobei sich jeweils die hydrophoben Reste der Ceramide direkt gegenüberliegen und in Kontakt treten. Sie bilden somit den hydrophoben inneren Bereich der Membrandoppelschicht, während die hydrophilen Reste auf beiden Seiten der Lipid-Doppelschicht mit der wässrigen Phase des Extra- und des Intrazellularraumes in Kontakt stehen. Die Tendenz zur Ausbildung dieser Ceramid-Doppelschicht besteht sowohl innerhalb als auch außerhalb eines Organismus, z. B. in einem wässrigen System, in welchem die Eigenschaften der Ceramid-Doppelschichten in eigens hierzu ausgelegten experimentellen Anordnungen untersucht werden können.

Bei allen neun bislang bekannten Ceramiden sowie bei den analogen Hydroxyceramiden besteht der lipophile Bereich aus dem Alkanrest der Sphingosin-Grundstruktur und dem an der NH-Gruppe des Sphingosins angekoppelten Fettsäurerest (Acylrest), während der hydrophile Bereich durch die beiden OH-Gruppen und durch die -NH-CO- Struktur des Sphingosin-Grundkörpers gebildet wird.

Die Struktur der Ceramid-Doppelschicht bildet sich in einem Organismus spontan aus. Obwohl sie eine erhebliche Stabilität besitzt, besteht z. B. bei Vorliegen einer Ceramid-Stoffwechselstörung die Möglichkeit, dass eine biologische Membran einen Teil ihrer Ceramid-Komponenten verliert, weil diese Komponenten entweder zu langsam und/oder in einem unzureichenden Ausmaß gebildet oder zu schnell verstoffwechselt werden. Dadurch verarmen die betreffenden Membranen an den jeweiligen Ceramid-Komponenten, was u. a. zu einer Störung der Membranstruktur und -funktion führt.

Die physiologische Zusammensetzung der Membran-Ceramide des Stratum corneum der menschlichen Haut ist allerdings noch aus einem zweiten Grund für die normale Struktur und Funktion der Haut von essentieller Bedeutung. Die Anwesenheit eines ausreichenden Gehalts an Ceramiden gewährleistet die uneingeschränkte Fähigkeit der Haut zur Bindung einer physiologischen Menge an Wasser. Der Verlust eines Teils der Stratum-corneum-Ceramide führt daher zu einer Einschränkung der Wasserbindefähigkeit, was durch die sog. Corneometrie klinisch feststellbar ist. Weiterhin erhöht sich im Zuge des Ceramidverlustes der sog. Transepidermale Wasserverlust (Trans Epidermal Water Loss = TEWL) der Haut. Dies zeigt sich im Auftreten einer "trockenen" und faltigen Haut, die insb. häufig, aber nicht ausschließlich, im höheren Lebensalter auftritt.

Ein bekanntes Beispiel für derartige Veränderungen ist die Verarmung der Lipid-Doppelschichten des Stratum corneum der menschlichen Haut an Ceramiden. Beispielsweise zeigten sich im Fall der Atopischen Dermatitis in der Haut der Patienten erniedrigte Gehalte u.a. an Ceramid 3, Ceramid 4, ω-Hydroxyceramiden (Macheleidt O, Kaiser HW und Sandhoff K (2002): J Invest Dermatol 119(1): 166-173) und Sphingosin.

Hautveränderungen und Hauterkrankungen als Folge des Verlustes von Lipiden, insb. von Ceramiden, sind beispielsweise:
- Atopische Dermatitis
- "trockene" Haut-Xerose, Xerodermie
- dyshidrotisches Ekzem
- chronisches kumulativ-toxisches Kontaktekzem
- alternde Haut
- durch UV-Licht stark beanspruchte Haut
- Sebostase
- Verhornungsstörungen
- Diabetes-bedingte Hautschäden

Insbesondere auf dem Gebiet der Klinischen Medizin ist es in den genannten Fällen von Erkrankungen wünschenswert, die Struktur der im Organismus vorhandenen biologischen Membran, i. e. der Lipid-Doppelschichten, in geeigneter Weise zu verändern und/oder zu stabilisieren.

Gemäß neueren Erkenntnissen zum Pathomechanismus der Atopischen Dermatitis (Arikawa J, Ishibashi M, Kawashima M, Takagi Y, Ichikawa Y, Imokawa G (2002): J Invest Dermatol, 119(2): 433-439) und verwandter Erkrankungen ist die Ursache für die Anfälligkeit der Haut im Fall einer derartigen Erkrankung u. a. ein veränderter Lipidstoffwechsel bzw. reduzierter Lipid-Gehalt des Stratum corneum. Diese Veränderungen betreffen neben dem Fettsäure-Stoffwechsel u.a. auch den Ceramid-Stoffwechsel.

Die heutigen Möglichkeiten, die Symptome und Folgen der genannten Hautkrankheiten, insb. der Atopischen Dermatitis, zu lindern (von einer Heilung kann derzeit noch keine Rede sein), sind nach wie vor noch sehr begrenzt. Die topische Anwendung spezieller Glucocorticoide und immunsuppressiver Wirkstoffe ist wegen der Toxizität dieser Substanzen mit erheblichen Risiken verbunden. Bestimmte Corticoide verursachen sogar einen geradezu kontraproduktiven Effekt, indem sie zu einem Verlust an Lipiden, insb. an Ceramiden, Cholesterol und freien Fettsäuren führen.

Unter Berücksichtigung des heutigen Kenntnisstands über die Bedeutung einer physiologischen LipidZusammensetzung der Stratum-corneum-Membranen ist es folgerichtig, dass versucht wird, die im Stratum corneum vorhandenen Defizite an Membran-Lipiden durch exogene Zufuhr auszugleichen. In der Praxis versucht man daher, mit Hilfe von Salben, Cremes und dergleichen die fehlenden Lipide, insb. Ceramide, der veränderten bzw. erkrankten Haut zuzuführen. Dies erfolgt beispielsweise durch speziell hierfür formulierte Lipid-Präparate, u. a. unter Verwendung von Liposomen als Vehikel zum Transport der Lipide in die Haut. Zahlreiche Produkte mit Gehalten an Ceramiden sind zu kosmetischen Zwecken und zur Therapie der genannten Hautkrankheiten inzwischen auf dem Markt.

Die hier geschilderten therapeutischen Maßnahmen sind sicherlich als prinzipiell richtig anzusehen, da sie in logischer Weise die vorhandenen Defizite des Stratum corneum an Ceramiden auszugleichen versuchen. Die während der letzten Jahre mit diesen therapeutischen Maßnahmen gemachten Erfahrungen zeigen jedoch, dass trotz der prinzipiellen Richtigkeit des therapeutischen Ansatzes die Ergebnisse dieser kosmetischen und der Heilbehandlungen keineswegs überzeugend sind. Zum Teil ist der Erfolg der durchgeführten Maßnahmen unsicher bzw. nicht nachhaltig. Selbst dann, wenn sich ein annähernd akzeptabler Erfolg der Heilbehandlung einstellt, hat eine derartige Behandlung zumindest zwei gravierende Nachteile:
- Das Ausmaß des Heilerfolgs ist nicht so groß, dass von einer vollständigen Gesundung der erkrankten Haut gesprochen werden kann.
- Um einen einigermaßen akzeptablen Heilerfolg an der Haut über einen längeren Zeitraum zu gewährleisten, müssen die Ceramide in kurzen Zeitabständen permanent der Haut zugeführt werden, d.h. die eingesetzten Wirksubstanzen zeigen keine nachhaltige Wirksamkeit.

Beide Nachteile sind auf eine gemeinsame Ursache zurückzuführen. Die Ceramide sind keine statischen Komponenten der Haut, sondern sie sind Zwischenprodukte einer Reaktionsfolge, in der die von der Haut benötigten Ceramide z.B. durch Synthesevorgänge des Organismus oder aus der Nahrung bereitgestellt und in die biologische Membran eingebaut werden. Nach Wahrnehmung ihrer Funktion als Membranbestandteil des Stratum corneum werden sie anschließend in spezifische Abbaureaktionen des Organismus eingeschleust.

Diese Reaktionsabfolge stellt ein Fließgleichgewicht dar, in welchem eine gewisse Menge an Ceramiden durch die Wirkung bestimmter Enzyme synthetisiert und nach einer entsprechenden Verweildauer in der Membran aus dieser wieder freigesetzt wird, um dann über enzymgesteuerte metabolische Abbauprozesse eliminiert zu werden. Es liegt somit ein gewisser Durchsatz an Substanz vor. Die als Hauttherapeutica exogen zugeführten Ceramide werden in diese Reaktionsabfolge eingeschleust. Liegt a priori eine Störung dieser Reaktionsabfolge vor, die dann zu einer pathologisch verminderten Ceramid-Zusammensetzung des Stratum corneum führt, so ist zu erwarten, dass die exogene Zufuhr von Ceramiden in Form eines Therapeuticums an diesem pathologischen Zustand grundlegend nichts oder nicht viel ändern kann, da der exogen zugeführte Ceramid-Anteil vom Organismus in gleicher Weise metabolisiert wird, wie dies bei dem endogen zur Verfügung gestellten Ceramid-Anteil der Fall ist.

Ein Heilerfolg ist daher mit den derzeit zur Verfügung stehenden therapeutischen Möglichkeiten in hohem Maße davon abhängig, dass die therapeutisch zugeführten Ceramide schneller in die Haut eindringen können als sie in die vorhandenen physiologischen Abbauschritte eingeschleust werden, und dass sie über einen längeren Zeitraum, im Extremfall lebenslang, kontinuierlich zugeführt werden.

Das vorliegende Problem ist nicht ohne weiteres lösbar. Der Geschwindigkeit der Aufnahme von Ceramiden in das Stratum corneum hinein sind gewisse physiologische und physikalisch-chemische bzw. biochemische Grenzen gesetzt, z. B. hinsichtlich der Diffusionsgeschwindigkeit der Ceramide. Diese Geschwindigkeit kann nicht beliebig gesteigert werden. Zum anderen sind die an den genannten Reaktionsfolgen beteiligten Ceramid-synthetisierenden und Ceramid-metabolisierenden Enzyme durch exogene Maßnahmen nicht oder nicht ohne gravierende Probleme im Sinne einer Erhöhung (synthetisierende Enzyme) oder einer Minderung ihrer Aktivität (metabolisierende Enzyme) zu beeinflussen.

Der erstgenannte therapeutische Ansatz, i.e., die Aktivierung lipidsynthetisierender Enzyme durch exogene Wirkstoffe (z.B. Nicotinamid), ist nur in begrenztem Umfang möglich und bisher lediglich *in vitro* gelungen (Tanno O, Ota Y, Kitamura N, Katsube T, Inoue S (2000): British J Dermatol, 143(3): 524-531). Der zweite therapeutische Ansatz, i.e., die Hemmung lipid-metabolisierender Enzyme durch exogene Wirkstoffe, ist bisher offenbar noch nicht gelungen, da offensichtlich Hemmstoffe Lipid-metabolisierender Enzyme mit ausreichend hoher Spezifität nicht existieren.

Zur Lösung des beschriebenen Problems ist es erforderlich, grundsätzlich andere Prinzipien anzuwenden, um die therapeutische Wirksamkeit exogen zugeführter Lipid-Ersatzsubstanzen oder -Analogsubstanzen zu erhöhen.

Die JP 2002-047261 beschreibt Diamid-Derivate, welche die Barrierefunktion und Wasserbindungsfunktion des Stratum corneum der Haut verbessern und eine Schutzwirkung für Haare aufweisen. Beispielhaft wird als Diamid-Derivat ein Ceramid-Dimer offenbart, wobei die beiden Ceramid-Monomere über ihr lipophiles Ende miteinander verknüpft sind.

Die Aufgabe der vorliegenden Erfindung ist es deshalb, Verbindungen bereitzustellen, durch welche
- die im Organismus vorhandenen biologischen Membranen stabilisiert werden können,
- die Wasserbindefähigkeit der verschiedenen Hautschichten erhöht werden kann und
- der transepidermale Wasserverlust (TEWL = Trans Epidermal Water Loss) der Haut vermindert werden kann.

Unter der Stabilisierung der biologischen Membran soll im vorliegenden Fall der Vorgang verstanden werden, dass die erfindungsgemäßen Wirksubstanzen bereitgestellt werden, die nach Einlagerung in die biologische Membran gegenüber aus dem Stand der Technik bekannten Ceramiden eine geringere Tendenz zum Verlassen der Membran zeigen, d.h. eine größere Nachhaltigkeit ihrer kosmetischen und klinischen Wirksamkeit besitzen.

Diese Aufgabe wird durch die Ceramid-Dimere mit den Merkmalen des Anspruchs 1 sowie hinsichtlich der Verwendung als Arzneimittel mit den Merkmalen des Anspruchs 4 oder 5 sowie hinsichtlich der Verwendung als kosmetische Zubereitung mit den Merkmalen des Anspruchs 7 gelöst. Die weiteren abhängigen Ansprüche zeigen vorteilhafte Weiterbildungen auf.

Erfindungsgemäß werden Ceramid-Dimere bereitgestellt, die aus zwei Ceramid-Molekülen aufgebaut sind, die über ihr lipophiles Ende miteinander verknüpft sind.

Dabei ist es unerheblich, ob die Bindung über die Alkylgruppe des Sphingosingrundkörpers oder über den Fettsäurerest oder über beide Gruppen bzw. Reste erfolgt.

Die Ceramid-Dimere weisen dabei jeweils am hydrophilen Ende mindestens eine hydrophile Gruppe zur Vergrößerung der Hydrathülle des Dimers auf.

Die erfindungsgemäßen Verbindungen aus der Gruppe der Ceramid-Dimere weisen folgende Eigenschaften auf:
- Die grundsätzliche Struktur der eingesetzten Ceramide, welche die Ausbildung einer Lipid-Doppelmembran ermöglicht, bleibt nicht nur erhalten, sondern die Fähigkeit zur Ausbildung der Doppelmembran wird verstärkt, weil die durch die genannten Erkrankungen geschädigte Haut ohnehin nur eine eingeschränkte Fähigkeit zum Aufbau und zum Erhalt der physiologischen Lipid-Doppelmembran besitzt.
- Die Struktur der eingesetzten Ceramide wird bei erhaltener Grundstruktur so verändert, dass sie nur noch in geringerem Umfang als Substrate der in der Haut, speziell im Stratum corneum, vorhandenen metabolisierenden Enzyme fungieren können. Dies bedeutet, dass sie in deutlich geringerem Umfang als die originären Ceramide in die jeweiligen enzymatischen Reaktionsfolgen eingeschleust werden und somit als wesentliche strukturelle Bestandteile des Stratum corneum über einen längeren Zeitraum als die originären Ceramide erhalten bleiben.
- Die Abänderung der speziellen Ceramid-Molekülstruktur erfolgt jedoch andererseits nur in einem so geringen Umfang, dass durch den geringen stoffwechselbedingten Um- oder Abbau der zugeführten Ceramide nur solche Substanzen entstehen, die den körpereigenen Ceramiden möglichst ähnlich sind. Auf diese Weise wird die Gefahr erheblich verringert, dass Stoffwechselprodukte mit toxischer Wirkung entstehen.
- Eine vergleichsweise geringe, steuerbare Abbaubarkeit der Ceramid-Dimere wird dadurch erreicht, dass die kovalente Bindung zwischen den beiden originären Ceramidmolekülen über ein Sauerstoffatom erfolgt. Dieses Sauerstoffatom wirkt als metabolische Sollbruchstelle, da z.B. mischfunktionelle Monooxygenasen die in unmittelbarer Nachbarschaft zum Sauerstoffatom stehenden Kohlenstoffatome oxidativ angreifen, was zu einem Auseinanderbrechen des Ceramid-Dimers unter Bildung der ω-hydroxylierten oder carboxylierten originären Ceramide führt.
- Durch die Anbindung eines weiteren Moleküls mit ausgeprägten hydrophilen Eigenschaften an den hydrophilen Molekülteil der Ceramide wird die Hydrathülle der Ceramid-Dimere vergrößert, was in der Folge zur Erhöhung der Wasserbindefähigkeit der betreffenden Hautschichten und zu einer Verminderung des transepidermalen Wasserverlustes (TEWL) führt.

Diese Eigenschaften werden erfindungsgemäß dabei folgendermaßen erreicht.

Durch die Kopplung zweier Ceramidmoleküle zu Ceramid-Dimeren ist die Gewähr gegeben, dass ein derartiges Molekül durch die im Organismus vorhandenen Enzyme des Ceramid-Stoffwechsels sehr viel langsamer ab- bzw. umgebaut wird, als dies für die originären Ceramide zutrifft. Die mit der kovalenten Bindung zwischen zwei Ceramidmolekülen verbundene Molekülvergrößerung führt zu einer starken Minderung der enzymatisch gesteuerten Metabolisierung, weil bei der bekanntermaßen hohen Substratspezifität der meisten Enzyme die (annähernde) Verdopplung der Größe des Ceramidmoleküls zu einem Ceramid-Dimer die Geschwindigkeit des Ceramidumsatzes bzw. des Ceramidabbaus erheblich abfallen lässt.

Andererseits sind die entstehenden Abbauprodukte von ihrem allgemeinen Aufbau her den natürlicherweise vorkommenden Folgeprodukten des Ceramid-Stoffwechsels so ähnlich, dass ein Einschleusen in die entsprechenden Reaktionsfolgen nach einem langsam ablaufenden Abbau der erfindungsgemäßen Wirksubstanzen ohne Probleme möglich ist. Darüber hinaus ist auch in keiner Weise damit zu rechnen, dass die Ceramid-Dimeren wegen der großen Ähnlichkeit mit den originären Ceramiden eine relevante Toxizität besitzen.

Ein gewisser Grad der enzymatischen Metabolisierung der Ceramid-Dimere, die allerdings als deutlich geringer anzusehen ist als die der monomeren Ceramide, ist somit eine aus pharmakokinetischen und pharmakodynamischen Gründen erwünschte Eigenschaft der Wirksubstanzen, weil hierdurch die Steuerbarkeit der Therapie besser gewährleistet ist, als wenn kein metabolischer Abbau möglich wäre.

Eine gewisse kontrollierte Metabolisierungsgeschwindigkeit der Ceramid-Dimere lässt sich dadurch erreichen, dass zwischen den beiden aneinandergekoppelten Ceramiden ein Sauerstoffatom vorhanden ist. Die in Nachbarschaft zu diesem Sauerstoffatom stehenden Kohlenstoffatome können enzymatisch hydroxyliert werden, etwa durch die Cytochrom-P₄₅₀-abhängigen mischfunktionellen Monooxygenasen. Eine derartige, in unmittelbarer Nachbarschaft zum Sauerstoffatom stattfindende Hydroxylierung führt zur Bildung instabiler Verbindungen mit Halbacetalstruktur, die in die entsprechenden Reaktionsprodukte zerfallen. Das eine Reaktionsprodukt ist ein Ceramidmolekül mit ω-ständiger OH-Gruppe, das andere Reaktionsprodukt ist ein Ceramidmolekül mit ω-ständiger Aldehydfunktion, die zur Carbonsäuregruppe weiteroxidiert wird. Damit wird offensichtlich, dass durch einen oxidativ ablaufenden biochemischen Abbau der beschriebenen Ceramid-Dimere Reaktionsprodukte entstehen, die den Ausgangsverbindungen der monomeren Ceramide sehr ähnlich sind.

Ein weiterer wesentlicher Aspekt der Pathogenese der oben genannten Hautveränderungen bzw. Hauterkrankungen ist die reduzierte Wasserbindefähigkeit des Hautgewebes, insbesondere im Bereich des Stratum corneum. Physiologischerweise wird das Wasser nicht innerhalb, sondern, da mehrere parallel angeordnete Lipid-Schichten vorliegen, in den Raum zwischen den einzelnen Lipid-Doppelschichten eingelagert. Dies liegt in der Tatsache begründet, dass das Innere der Lipid-Doppelschicht aus den stark hydrophoben Fettsäureresten z.B. der Ceramide aufgebaut ist, während das Medium außerhalb der Lipid-Doppelschicht hydrophiler Natur ist. Eine Speicherung von Wasser in den hydrophoben inneren Bereichen der Lipid-Doppelmembran ist praktisch nicht möglich.

Die anfangs genannten Hautveränderungen und -erkrankungen sind zum einen auf den Verlust eines Teiles der Lipide, insb. der Ceramide, aus den parallel angeordneten Lipid-Doppelschichten, zum anderen auf den partiellen Verlust von Wasser aus den zwischen diesen Doppelschichten angeordneten hydrophilen Zwischenschichten zurückzuführen. Ziel der therapeutischen Maßnahmen bei diesen Erkrankungen ist somit nicht nur der Wiederaufbau und die Stabilisierung der Lipid-Doppelschichten selbst, wie dies mit Hilfe der oben beschriebenen Ceramid-Dimere erfolgt, sondern weiterhin auch der Aufbau und die Stabilisierung einer ausgeprägten Hydrosphäre an der Oberfläche der Lipid-Doppelschicht, die für die Wasserbindefähigkeit der Haut von ausschlaggebender Bedeutung sind.

Zwar wird eine gewisse Menge Wasser an den Lipid-Doppelschichten gebunden und trägt somit zum natürlichen Wasserreservoir der Haut bei. Bei der alternden oder erkrankten Haut reicht diese Wasserbindefähigkeit jedoch offensichtlich nicht mehr aus, um die natürliche Struktur der Haut zu erhalten. Es ist daher erforderlich, diese Wasserbindungskapazität der Lipid-Doppelschichten dadurch zu erhöhen, dass an den hydrophilen Molekülteilen der beschriebenen Ceramid-Dimere zusätzlich hydrophile Gruppen angekoppelt werden.

Dabei ist es unerheblich, ob die Bindung über die Hydroxylgruppe oder die Hydroxymethylengruppe des Sphingosingrundkörpers erfolgt.

Hydrophile Gruppen mit einer gesteigerten Tendenz zur Bindung von Wasser sind stark polare Verbindung, insb. mit positiven oder negativen Ladungen und/oder mit solchen funktionellen Gruppen, die Wasser über Wasserstoffbrückenbindungen anzulagern vermögen. Hierzu gehören insbesondere

Aminosäuren, die stark polare Gruppen oder Ladungen besitzen: -COOH, -NH₂, -COO⁻ und -NH₃⁺, bevorzugt Serin, Threonin, Lysin, Arginin, Histidin, Asparaginsäure, Glutaminsäure, Asparagin, Glutamin und Tyrosin.

Polyole, wie Ethandiol oder Glycerin (Propantriol) mit mehreren -OH-Gruppen im Molekül, die zur Ausbildung von Wasserstoffbrückenbindungen befähigt sind.

Zucker wie Glucose oder Galaktose, bei denen mehrere OH-Gruppen im Molekül vorhanden sind.

Zucker-Derivate, wie Glucuronsäure oder Galakturonsäure mit mehreren OH-Gruppen und einer dissoziierbaren -COOH-Gruppe
Zucker-Derivate, wie Aminozucker, die Bestandteile der stark wasserbindenden Hyaluronsäure darstellen.

Organische Säuren, wie die Di- bzw. Tricarbonsäuren Malonsäure, Bernsteinsäure, Äpfelsäure oder Citronensäure, deren nicht für die Bindung an das Lipidmolekül gebrauchten weiteren Carboxylreste dissoziiert und daher geladen vorliegen, was mit einer hohen Wasserbindefähigkeit einhergeht.

Anorganische Säuren, wie z.B. Sulfat und Phosphat, die auf Grund ihrer im Molekül vorhandenen Ladungen eine sehr hohe Wasserbindefähigkeit besitzen. Cholesterolsulfat kommt beispielsweise als natürlicher Bestandteil biologischer Membranen vor.

Cholin als physiologische Substanz mit einem (positiv geladenen) quarternären N-Atom.

Derivate des Harnstoffs: Diese Verbindung (auch im Deutschen unter der englischen Bezeichnung "urea" geführt) ist in freier Form bereits heute Bestandteil vieler Salben, mit denen eine Erhöhung der Wasserbindefähigkeit der Haut bewirkt werden soll.

Erfindunsgemäß ist die hydrophile Gruppe ausgewählt aus der Gruppe bestehend aus Aminosäuren, Polyole, Zucker, organische Säuren, Cholin, Harnstoff oder Harnstoffderivate, sowie Kombinationen hiervon. Erfindungsgemäß weisen die Ceramid-Moleküle die allgemeinen Formeln I bis III auf der Basis des Sphingosins, des 6-Hydroxysphingosins und des Phytosphingosins auf:
Sphingosin-Grundkörper 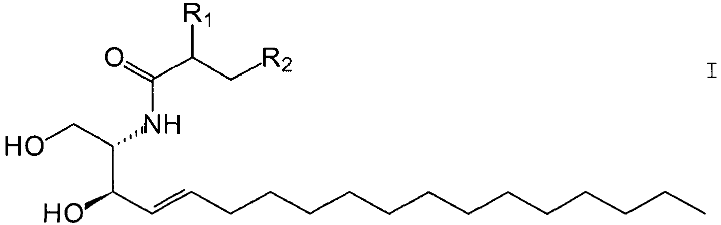
Hydroxysphingosin-Grundkörper 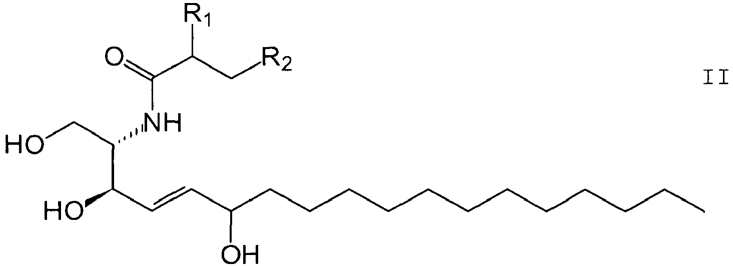
Phytosphingosin-Grundkörper 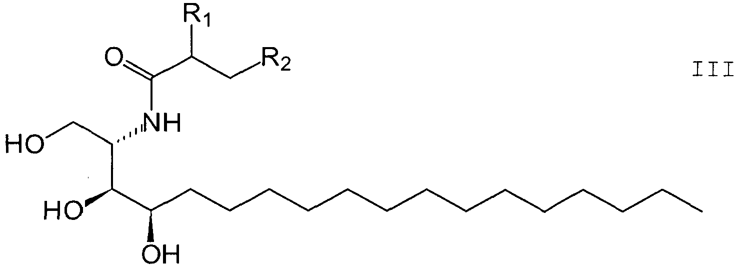 jeweils mit R₁ = H oder OH und R₂ = verzweigter oder geradkettiger C₁-C₁₆-Alkylrest, der gegebenenfalls mit Heteroatomen substituiert sein kann, oder ω-Hydroxyalkylrest mit Linolensäure verestert.

Die hierfür eingesetzten Ceramide bestehen dabei bevorzugt aus den bisher in der wissenschaftlichen Literatur beschriebenen Verbindungen Ceramid-1 (Ceramid EOS), Ceramid-2 (Ceramid NS), Ceramid-3 (Ceramid NP), Ceramid-4 (Ceramid EOH), Ceramid-5 (Ceramid AS), Ceramid-6 (Ceramid AP), Ceramid-7 (Ceramid AH), Ceramid-8 (Ceramid NH) und Ceramid-9 (Ceramid EOP) sowie den entsprechenden ω-Hydroxyceramiden. Die genannten Ceramide stellen allerdings keine einheitlichen Substanzen mit genau definierter relativer Molmasse dar, sondern jedes Ceramid stellt eine Familie von Verbindungen mit unterschiedlicher Länge der im Molekül vorhandenen amidartig gebundenen Fettsäure und/oder des im Sphingosinanteil vorhandenen Alkylrestes dar.

Die Ceramid-Moleküle sind erfindungsgemäß über ihr lipophiles Ende kovalent über einen Spacer miteinander verbunden. Der Spacer besteht dabei aus einem Sauerstoffatom oder einer -O-(CH₂)ₙ-O-Gruppe mit n = 1 bis 20.

Erfindungsgemäß wird ebenso das zuvor beschriebene Ceramid-Dimer zur Verwendung als Arzneimittel bereitgestellt.

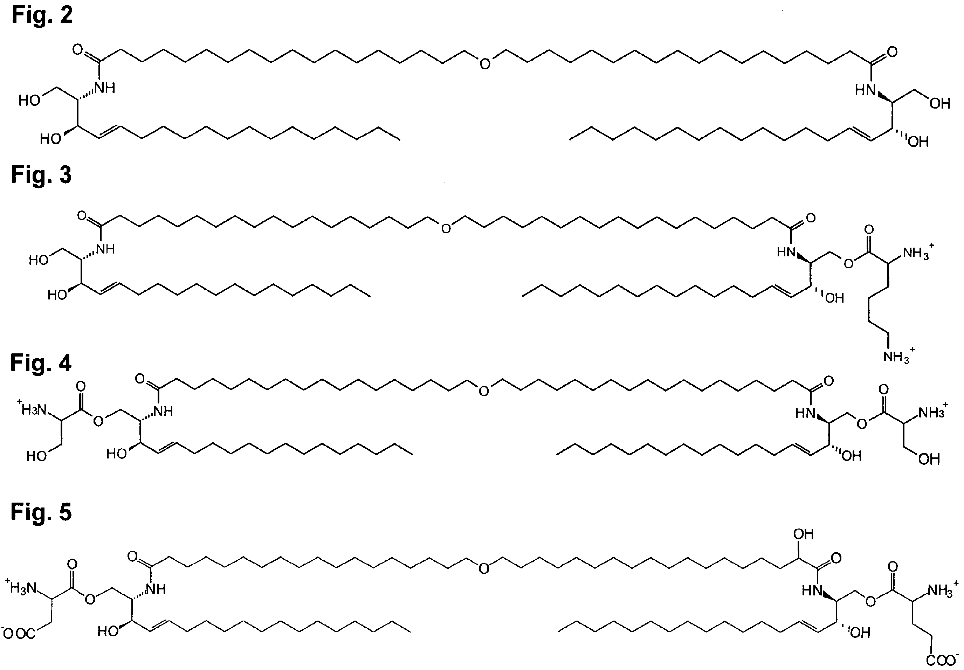

Fig. 3 zeigt eine Wirksubstanz, in der die Lipid-Komponente aus einem Dimer des Ceramid 2 besteht. Zur Erhöhung der Hydrophilie der an der Membranoberfläche liegenden hydrophilen Molekülareale ist an einer der beiden Ceramid-2-Komponenten ein Lysinrest angekoppelt.

Erfindungsgemäß wird das Ceramid-Dimer, wie es zuvor beschrieben wurde, zur Herstellung eines Arzneimittels, zur Behandlung von Erkrankungen, bei denen eine Störung der Lipidzusammensetzung der Zellmembranen eines Organismus hinsichtlich ihres Gehalts an Ceramiden und Sphingosinen vorliegt, bereitgestellt.

Das zuvor beschriebene Ceramid-Dimer kann auch zur Herstellung eines Arzneimittels zur Behandlung von Erkrankungen, bei denen eine Störung der Zusammensetzung der Lipid-Doppelschichten des Stratum corneum der Haut hinsichtlich ihres Gehaltes an Ceramiden und Sphingosinen vorliegt, eingesetzt werden.

Eine weitere Verwendung der erfindungsgemäßen Ceramid-Dimere betrifft die Herstellung von kosmetischen Zubereitungen, insbesondere als Creme, Salbe, Lotion, Emulsion, Gel, Spray, kosmetisches Öl oder Liposomen.

Anhand der nachfolgenden Figuren und Beispiele soll der erfindungsgemäße Gegenstand näher erläutert werden, ohne diesen auf die hier gezeigten speziellen Ausführungsformen einschränken zu wollen.

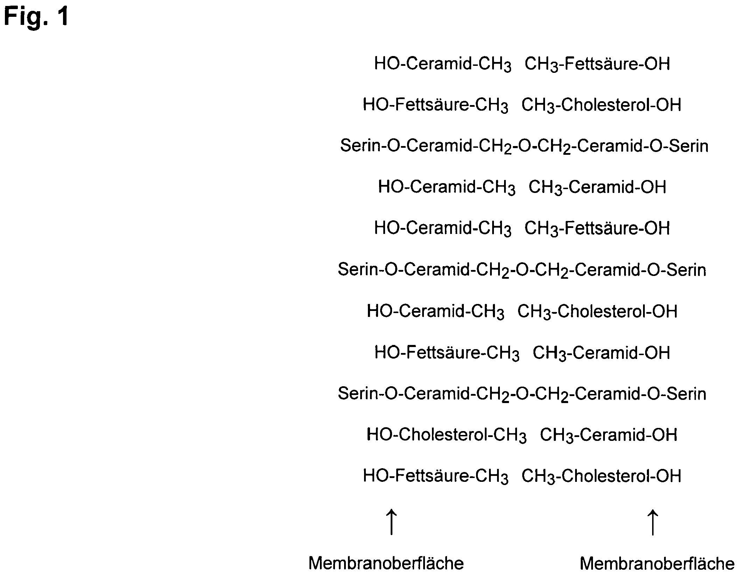

Fig. 1 zeigt den schematischen Aufbau biologischer Membranen des Stratum corneum aus den Hauptlipiden Ceramide, Cholesterol und freien Fettsäuren mit der zusätzlichen Einlagerung von Ceramid-Dimeren mit zwei angekoppelten Serin-Resten.
Fig. 2 zeigt eine Wirksubstanz, in der die Lipid-Komponente aus einem Dimer des Ceramid 2 besteht.
Fig. 4 zeigt eine erfindungsgemäße Wirksubstanz, in der die Lipid-Komponente aus einem Dimer des Ceramid 2 besteht. Zur Erhöhung der Hydrophilie der an der Membranoberfläche liegenden hydrophilen Molekülareale ist an den beiden Ceramid-2-Komponenten jeweils ein Serinmolekül angekoppelt.
Fig. 5 zeigt eine erfindungsgemäße Wirksubstanz, in der die Lipid-Komponente aus einem Molekül Ceramid 2 und einem Molekül Ceramid 5 besteht. Zur Erhöhung der Hydrophilie der an der Membranoberfläche liegenden hydrophilen Molekülareale sind an den beiden Ceramid-Komponenten ein Glutaminsäurerest und ein Asparaginsäurerest angekoppelt.

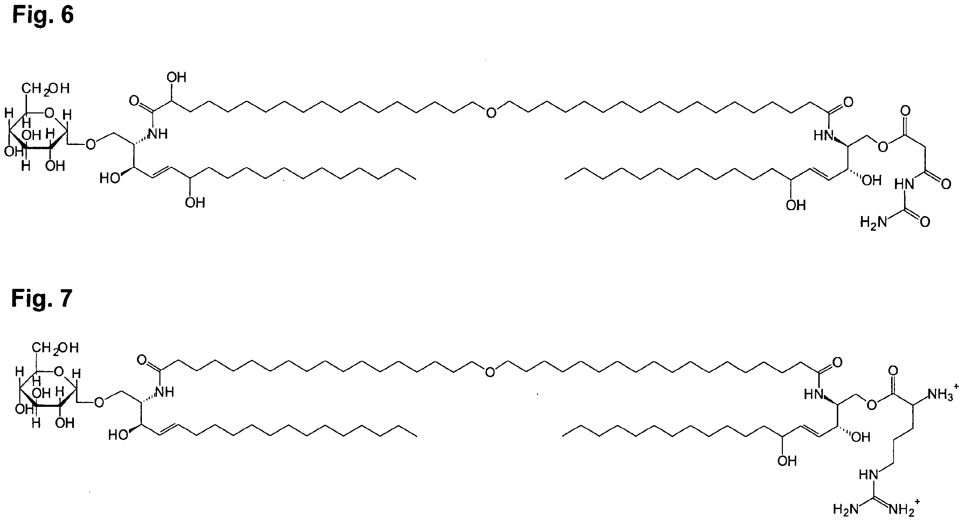

Fig. 6 zeigt eine erfindungsgemäße Wirksubstanz, in der die Lipid-Komponente aus einem Molekül Ceramid 7 und einem Molekül Ceramid 8 besteht. Zur Erhöhung der Hydrophilie der an der Membranoberfläche liegenden hydrophilen Molekülareale ist am Ceramid 7 ein Glucoserest und am Ceramid 8 ein Harnstoffderivat (einen Malonsäurerest enthaltend) angekoppelt.
Fig. 7 zeigt eine erfindungsgemäße Wirksubstanz, in der die Lipid-Komponente aus einem Molekül Ceramid 2 und einem Molekül Ceramid 8 besteht. Zur Erhöhung der Hydrophilie der an der Membranoberfläche liegenden hydrophilen Molekülareale ist am Ceramid 2 ein Glucoserest und am Ceramid 8 ein Arginin-Rest angekoppelt.

### Beispiel

Gemäß den fachärztlichen dermatologischen Gutachten der Dermatest(R) GmbH, 48143 Münster, zeigen die beiden Wirkstoffe

HO-Ceramid-CH₂-O-CH₂-Ceramid-OH

("Dimer", entspr. Fig. 2)
und

Serin-O-Ceramid-CH₂-O-CH₂-Ceramid-O-Serin

("Serin-Dimer", entspr. Fig. 4)
mit Ceramid-2 als Ceramidkomponente hinsichtlich der Corneometrie (Wasserbindevermögen) und TEWL (Wasserverlust) bei 10 Probanden mit Neurodermitis und 10 Probanden mit Xerodermie nach 1, 2 und 4 Wochen die folgenden Ergebnisse (alle Angaben in % Veränderung gegenüber dem Ausgangswert):

### Dimer (entspr. Fig. 2):

### Corneometrie (Wasserbindevermögen):

| | 1 Woche | 2 Wochen | 4 Wochen |
|---|---|---|---|
| Alle 20 Probanden | + 24,05 | + 28,02 | + 37,60 |
| 10 Prob. Neurodermitis | + 25,63 | + 28,62 | + 43,40 |
| 10 Prob. Xerodermie | + 22,52 | + 27, 42 | + 31,90 |

### TEWL (Wasserverlust):

| | 1 Woche | 2 Wochen | 4 Wochen |
|---|---|---|---|
| Alle 20 Probanden | - 8,65 | - 13,32 | - 16,75 |
| 10 Prob. Neurodermitis | - 7,81 | - 13,50 | - 17,02 |
| 10 Prob. Xerodermie | - 9,46 | - 13,04 | - 16,39 |

Beim TEWL-Wert wird die Minderung des Wasserverlustes erfasst. Dies bedeutet, dass die Wirksamkeit der erfindungsgemäßen Wirksubstanz umso höher ist, je stärker negativ der Messwert ist.

### Serin-Dimer (s. Fig. 4):

### Corneometrie (Wasserbindevermögen):

| | 1 Woche | 2 Wochen | 4 Wochen |
|---|---|---|---|
| Alle 20 Probanden | + 25,43 | + 29,49 | + 40,87 |
| 10 Prob. Neurodermitis | + 27,71 | + 32,88 | + 47,43 |
| 10 Prob. Xerodermie | + 23,23 | + 26,21 | + 34,53 |

### TEWL (Wasserverlust):

| | 1 Woche | 2 Wochen | 4 Wochen |
|---|---|---|---|
| Alle 20 Probanden | - 7,80 | - 12,57 | - 15,37 |
| 10 Prob. Neurodermitis | - 6,20 | - 9,74 | - 12,84 |
| 10 Prob. Xerodermie | - 9,36 | - 15,45 | - 17,93 |

Die Ergebnisse der durchgeführten dermatologischen Untersuchungen besagen, dass beide Wirksubstanzen eine gute bis sehr gute Wirksamkeit hinsichtlich der (erwünschten) Erhöhung des Wasserbindevermögens der Haut und hinsichtlich der (erwünschten) Erniedrigung des Wasserverlustes aus der Haut besitzen.

Eine differenziertere Bewertung der Ergebnisse zeigt, dass das Serin-Dimer stärker als das Dimer das Wasserbindevermögen der Haut begünstigt. Dieser Effekt tritt besonders beim Vorliegen von Neurodermitis auf.

Andererseits begünstigt das Dimer etwas stärker als das Serin-Dimer den Wasserverlust aus der Haut im Fall der Neurodermitis.

## Patentansprüche

1. Ceramid-Dimer aus zwei Ceramid-Molekülen, wobei die Ceramid-Moleküle einen Sphingosin-Grundkörper, Hvdroxvsphingosin-Grundkörper oder einen Phytosphingosin-Grundkörper gemäß einer der allgemeinen Formeln I bis III aufweisen 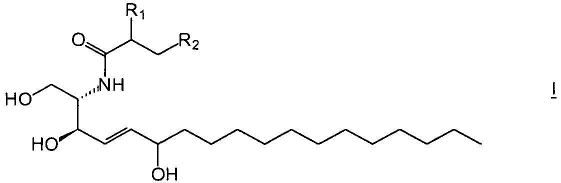 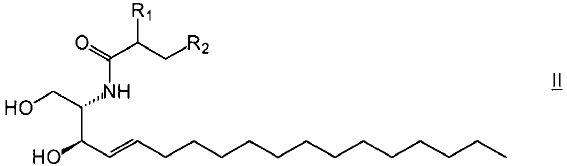 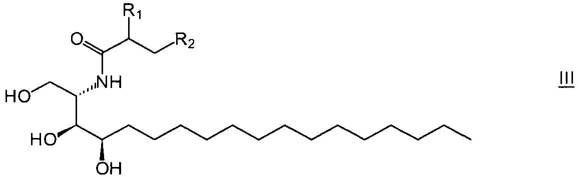 jeweils mit R₁ = H oder OH und R₂ = verzweigter oder geradkettiger C₁-C₁₆-Alkylrest, der gegebenenfalls mit Heteroatomen substituiert sein kann, oder ω-Hydroxyalkylrest mit Linolensäure verestert,
wobei die zwei Ceramid-Moleküle über ihr lipophiles Ende kovalent über einen Spacer miteinander verknüpft sind, entweder über die Alkylkette der Sphingosin-Grundstruktur oder über den Fettsäurerest oder über die Kombination beider Angriffspunkte, wobei der Spacer aus einem Sauerstoffatom oder einer -O-(CH₂)ₙ-O-Gruppe mit n = 1 bis 20 besteht,
**dadurch gekennzeichnet, dass** am hydrophilen Ende der Ceramid-Moleküle über die Hydroxylgruppe oder die Hydroxymethylengruppe des Sphingosingrundkörpers mindestens eine hydrophile Gruppe zur Vergrößerung der Hydrathülle des Dimers angeordnet ist und die hydrophile Gruppe ausgewählt ist aus der Gruppe bestehend aus Aminosäuren, Polyole, Zucker, organische Säuren, Cholin, Harnstoff oder Harnstoffderivate, sowie Kombinationen hiervon.

2. Ceramid-Dimer nach Anspruch 1,
**dadurch gekennzeichnet, dass** die hydrophile Gruppe ausgewählt ist aus der Gruppe bestehend aus
Aminosäuren mit polaren Gruppen oder Ladungen, bevorzugt ausgewählt aus der Gruppe bestehend aus -COOH, -NH₂, -COO⁻ und -NH₃⁺, bevorzugt Serin, Threonin, Lysin, Arginin, Histidin, Asparaginsäure, Glutaminsäure, Asparagin, Glutamin, Tyrosin und Tryptophan, Ethandiol oderGlycerin,
Glucose oder Galaktose, Zuckerderivate mit einer dissoziierbaren-COOH-Gruppe, insbesondere Glucuronsäure oder Galakturonsäure, oder Aminozucker,
Di-bzw. Tricarbonsäuren, bevorzugt Malonsäure, Bernsteinsäure, Äpfelsäure oder Citronensäure,
anorganischen Säuren, insbesondere Schwefel- oder Phosphorsäuren, sowie Kombinationen hiervon.

3. Ceramid-Dimer nach dem vorhergehenden Anspruch,
**dadurch gekennzeichnet, dass** die Ceramid-Moleküle ausgewählt sind aus der Gruppe bestehend aus Ceramid-1, Ceramid-2, Ceramid-3, Ceramid-4, Ceramid-5, Ceramid-6, Ceramid-7, Ceramid-8, Ceramid-9 sowie deren w-Hydroxyceramiden.

4. Ceramid-Dimer nach einem der vorhergehenden Ansprüche zur Verwendung als Arzneimittel.

5. Ceramid-Dimer nach einem der Ansprüche 1 bis 3 zur Herstellung eines Arzneimittels zur Behandlung von Erkrankungen, bei denen eine Störung der Lipidzusammensetzung der Zellmembranen eines Organismus hinsichtlich ihres Gehalts an Ceramiden, w-Hydroxyceramiden und Sphingosinen vorliegt.

6. Ceramid-Dimer nach einem der Ansprüche 1 bis 3 zur Herstellung eines Arzneimittels zur Behandlung von Erkrankungen, bei denen eine Störung der Zusammensetzung der Lipid-Doppelschichten des Stratum corneum der Haut hinsichtlich ihres Gehaltes an Ceramiden, w-Hydroxyceramiden und Sphingosinen vorliegt.

7. Verwendung der Ceramid-Dimere nach einem der Ansprüche 1 bis 3 zur Herstellung einer kosmetischen Zubereitung, insbesondere als Creme, Salbe, Lotion, Emulsion, Gel, Spray, kosmetisches Öl oder Liposomen.

## Claims

1. A ceramide dimer comprising two ceramide molecules, wherein the ceramide molecules have a sphingosine basic body, hydroxysphingosine basic body or a phytosphingosine basic body according to one of the general formulas I to III 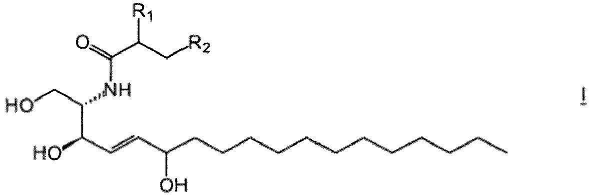 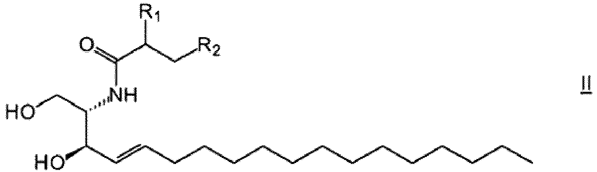 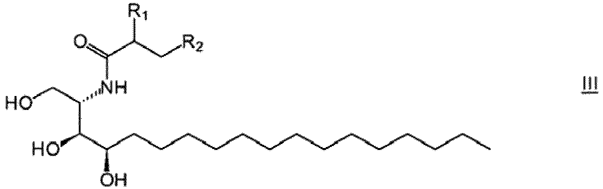 wherein, in each case, R₁ = H or OH, and R₂ = branched or straight-chain C₁-C₁₆ alkyl group, which optionally can be substituted with heteroatoms, or w-hydroxyalkyl group esterified with linoleic acid,
wherein the two ceramide molecules are covalently bonded to each other via their lipophilic end, either via the alkyl chain of the sphingosine basic body or via the fatty acid group or via the combination of both attack points, wherein the spacer consists of an oxygen atom or an -O-(CH₂)ₙ-O group where n = 1 to 20,
**characterized in that** at least one hydrophilic group is disposed at the hydrophilic end of the ceramide molecules, via the hydroxyl group or via the hydroxymethylene group of the sphingosine basic body, for increasing the hydration shell of the dimer, and the hydrophilic group is selected from the group consisting of amino acids, polyols, sugars, organic acids, choline, urea or urea derivatives, and combinations thereof.

2. The ceramide dimer according to claim 1,
**characterized in that** the hydrophilic group is selected from the group consisting of amino acids having polar groups or charges, preferably selected from the group consisting of -COOH, -NH₂, -COO⁻ and -NH₃⁺, preferably serine, threonine, lysine, arginine, histidine, aspartic acid, glutamic acid, asparagine, glutamine, tyrosine, and tryptophan,
ethane diol or glycerol,
glucose or galactose, sugar derivatives having a dissociable -COOH group, in particular glucuronic acid or galacturonic acid, or amino sugar,
di- or tricarboxylic acids, preferably malonic acid, succinic acid, malic acid or citric acid,
inorganic acids, in particular sulphuric or phosphoric acids, and combinations thereof.

3. The ceramide dimer according to the preceding claim,
**characterized in that** the ceramide molecules are selected from the group consisting of ceramide 1, ceramide 2, ceramide 3, ceramide 4, ceramide 5, ceramide 6,
ceramide 7, ceramide 8, ceramide 9 and the ω-hydroxyceramides thereof.

4. The ceramide dimer according to any one of the preceding claims for use as a pharmaceutical.

5. The ceramide dimer according to any one of claims 1 to 3, for producing a pharmaceutical for treating diseases in which a disorder of the lipid composition of the cell membranes of an organism with respect to its content of ceramides, ω-hydroxyceramides and sphingosines is present.

6. The ceramide dimer according to any one of claims 1 to 3, for producing a pharmaceutical for treating diseases in which a disorder of the composition of the lipid bilayers of the stratum corneum of the skin with respect to its content of ceramides, ω-hydroxyceramides and sphingosines is present.

7. Use of the ceramide dimer according to any one of claims 1 to 3 for producing a cosmetic preparation, in particular a cream, an ointment, a lotion, an emulsion, a gel, a spray, a cosmetic oil or liposomes.

## Revendications

1. Dimère de céramide constitué de deux molécules de céramide, les molécules de céramide comportant un corps de base sphingosine, un corps de base hydroxysphingosine ou un corps de base phytosphingosine selon l'une des formules générales I à III 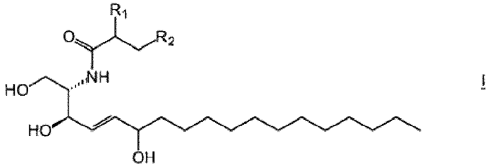 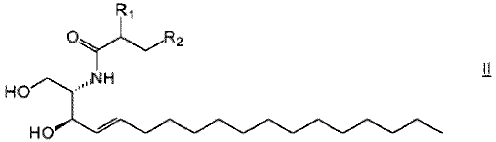 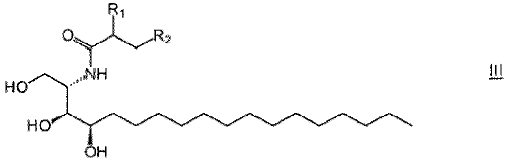 dans chacune desquelles R₁ = H ou OH, et R₂ est un radical alkyle en C₁-C₁₆ à chaîne droite ou ramifiée, qui peut éventuellement être substitué par des hétéroatomes, ou un radical ω-hydroxyalkyle estérifié avec l'acide linolénique,
les deux molécules de céramide étant reliées l'une à l'autre par leur extrémité lipophile par une liaison covalente par l'intermédiaire d'un espaceur, soit par l'intermédiaire de la chaîne alkyle de la structure de base sphingosine, soit par l'intermédiaire du radical acide gras, soit par l'intermédiaire de la combinaison des deux points d'application, l'espaceur étant constitué d'un atome d'oxygène ou d'un groupe -O-(CH₂)ₙ-O, dans lequel n = 1 à 20,
**caractérisé en ce que**, au niveau de l'extrémité hydrophile de la molécule de céramide, par l'intermédiaire du groupe hydroxyle ou du groupe hydroxyméthylène du corps de base sphingosine, au moins un groupe hydrophile est disposé pour augmenter l'enveloppe hydrate du dimère, et que le groupe hydrophile est choisi dans le groupe consistant en les acides aminés, les polyols, les sucres, les acides organiques, la choline, l'urée ou les dérivés de l'urée, ainsi que les combinaisons de ceux-ci.

2. Dimère de céramide selon la revendication 1, **caractérisé en ce que** le groupe hydrophile est choisi dans le groupe consistant en
les acides aminés comportant des groupes polaires ou des charges, de préférence choisis dans le groupe consistant en -COOH, -NH₂, -COO⁻ et -NH₃⁺, de préférence la sérine, la thréonine, la lysine, l'arginine, l'histidine, l'acide asparagique, l'acide glutamique, l'asparagine, la glutamine, la tyrosine et le tryptophane,
l'éthanediol ou le glycérol,
le glucose ou le galactose, les dérivés du sucre présentant un groupe COOH dissociable, en particulier l'acide glucuronique ou l'acide galacturonique, ou les amino-sucres,
les acides di- ou tricarboxyliques, de préférence l'acide malonique, l'acide succinique, l'acide malique ou l'acide citrique,
les acides inorganiques, en particulier les acides sulfurique ou phosphorique, ainsi que les combinaisons de ceux-ci.

3. Dimère de céramide selon la revendication précédente, **caractérisé en ce que** la molécule de céramide est choisie dans le groupe consistant en le céramide-1, le céramide-2, le céramide-3, le céramide-4, le céramide-5, le céramide-6, le céramide-7, le céramide-8, le céramide-9, ainsi que leurs ω-hydroxycéramides.

4. Dimère de céramide selon l'une des revendications précédentes, destiné à une utilisation en tant que médicament.

5. Dimère de céramide selon l'une des revendications 1 à 3, destiné à la fabrication d'un médicament pour le traitement de maladies dans lesquelles on est en présence d'une perturbation de la composition lipidique des membranes cellulaires d'un organisme, pour ce qui concerne sa teneur en céramides, en ω-hydroxycéramides et en sphingosines.

6. Dimère de céramide selon l'une des revendications 1 à 3, destiné à la fabrication d'un médicament pour le traitement de maladies dans lesquelles on est en présence d'une perturbation de la composition des doubles couches lipidiques de la couche cornée de la peau, pour ce qui est de leur teneur en céramides, en ω-hydroxycéramides et en sphingosines.

7. Utilisation des dimères de céramide selon l'une des revendications 1 à 3, pour la fabrication d'une préparation cosmétique, en particulier sous forme de crème, de pommade, de lotion, d'émulsion, de gel, de spray, d'huile cosmétique ou de liposomes.
